Europäisches Patentamt

European Patent Office

Office européen des brevets

(19)

(11) Veröffentlichungsnummer: **0 226 740**

**A1**

(12)  # EUROPÄISCHE PATENTANMELDUNG

(21) Anmeldenummer: **86114175.2**

(22) Anmeldetag: **14.10.86**

(51) Int. Cl.⁴: **A 61 B 17/22**

(30) Priorität: **14.12.85 DE 3544344**

(43) Veröffentlichungstag der Anmeldung:
**01.07.87 Patentblatt 87/27**

(84) Benannte Vertragsstaaten:
**CH FR GB LI**

(71) Anmelder: **DORNIER MEDIZINTECHNIK GMBH**
**Postfach 1128**
**D-8034 Germering 1(DE)**

(72) Erfinder: **Schwarze, Werner, Dr.**
**Johanniter Strasse 4**
**D-7768 Stockach 13(DE)**

(72) Erfinder: **Brendel, Walter, Prof. Dr.**
**Egenhofstrasse 17a**
**D-8033 Planegg(DE)**

(74) Vertreter: **Landsmann, Ralf, Dipl.-Ing.**
**Kleeweg 3**
**D-7990 Friedrichshafen 1(DE)**

(54) **Vorrichtung zur Thromboisierung mittels Stosswellen.**

(57) Mittels fokussierter Stosswellen, deren Energie zur Zerstörung von Zellen nicht ausreicht werden Veränderungen in den Wänden von Blutgefässen zur Versorgung von Tumorgewebe erzeugt. Dadurch sollen sich in diesen Blutgefässen Thromben bilden, wodurch das Tumorwachstum gehemmt wird.

Fig. 5

EP 0 226 740 A1

DORNIER MEDIZINTECHNIK GMBH
Postfach 1128
8034 Germering 1

0226740

Reg. M 105 EU

## THROMBOISIERUNG MITTELS STOSSWELLEN

Die Erfindung betrifft ein Verfahren und eine Vorrichtung zur Durchführung des Verfahrens zur Thromboisierung mittels Stoßwellen.

Es ist bekannt, mittels Stoßwellen im Körper von Lebewesen befindliche Nierensteine zu zertrümmern. Dies beruht auf folgenden physikalischen Grundlagen: treffen Stoßwellen auf Grenzflächen, an denen sich die Schallimpendanz unstetig ändert, so werden sie entsprechend der akustischen Eigenschaften dieser Grenzfläche als Druckwellen oder Zugwellen reflektiert. Wird dabei die Druckfestigkeit oder Zugfestigkeit des Materials überschritten, kommt es zur mechanischen Zerstörung. Zur Zertrümmerung von Nierensteinen werden fokussierende Methoden angewandt, um die Belastung des von der Stoßwelle zu durchlaufenden Gewebes gering zu halten und im Bereich des Nierensteins eine hohe Energiekonzentration zu bekommen.

Befindet sich im Fokus der Stoßwelle anstelle eines Steins Gewebe, so kann dieses durch die Stoßwelle zerstört werden. Dieser Effekt kann zur Zerstörung von gutartigen Tumoren verwendet werden, weil bei solchen Tumoren nicht die Gefahr besteht, daß Metastasen auftreten. Bei bösartigen Tumoren (Krebszellen) ist eine Zerstörung der Krebszellen möglich, wobei jedoch beachtet werden muß, daß zur Vermeidung des Auftretens von Metastasen die Zellen weit bis in den gesunden Bereich hinein zerstört werden müssen. Außerdem besteht die Gefahr, daß nur teilweise zerstörte Zellen Metastasen bilden, in dem sie an Ort und Stelle neu beginnen zu wuchern oder über das Blutgefäßsystem auf andere Körperteile verstreut werden und sich dort ansiedeln (Fernmetastasen).

Der Erfindung liegt die Aufgabe zugrunde, das Wachstum und die weitere Ausbreitung von pathologischen Neubildungen (Tumoren) im Körper zu verhindern:

Diese Aufgabe wird erfindungsgemäß dadurch gelöst, daß fokussierte Stoßwellen, deren Energie unterhalb des Zerstörungseffektes von Zellen liegt, auf die die pathologische Neubildung versorgenden Blutgefäße einwirken, dort Gefäßwandveränderungen hervorrufen, die wiederum zur Thromboisierung der betreffenden Blutgefäße führen.

Größere Gefäße und Blutgefäße gesunder Gewebe haben im Gegensatz zu Blutgefäßen, welche Tumorgewebe versorgen, dickere Wände, nämlich sowohl Endothelschicht als auch Basalmembran und bei Arteriolen und größeren Venolen auch eine Muskelschicht. Solche Gefäße werden durch Stoßwellen geeigneter therapeutischer Intensität kaum bis gar nicht verändert.

Die versorgenden Blutgefäße von Tumorgewebe haben meistens nur eine aus einer Endothelschicht bestehende dünne Wand und sind deshalb durch ihre spezielle anatomische Beschaffenheit gegenüber Stoßwellen bedingten Veränderungen besonders empfinglich. Durch fokussierende Stoßwellen wird erfindungsgemäß die Wand der einen Tumor versorgenden Kapillaren Veränderungen unterworfen, dodaß in diesen Gefäßarealen Mikrothromben entstehen. Das Wachstum von Metastasen wird gehemmt, da die den Tumor versorgenden kleinen Blutgefäße in ihrer Funktion gestört werden. Mikrothromben können in einem ebenfalls die beschriebene Wirkung unterstützenden kummulativen Prozeß zur Thromboisierung der ableitenden Venolen führen.

Zur Unterstützung der Wirkung sind folgende zusätzlich angewandte Verfahren denkbar:

- Lokale Hyperthermie der pathologischen Neubildung bei gleichzeitiger Stoßwellenbehandlung.

- Gabe von Medikamenten, die die Koagulation des Blutes während der Stoßwellenbehandlung fördern.

- Gabe von zytostatischen Substanzen, die bedingt durch die erhöhte Membranpermeabilität nach der Stoßwellenbehandlung verstärkt in die beschallten Zellen aufgenommen werden, was die Wirkung der Zytostatika erhöht.

Bei einer vorteilhaften Ausführungsform der Erfindung erfolgt die Beaufschlagung der Blutgefäße der pathologischen Neubildung aus verschiedenen Richtungen (Winkeln). Dadurch wird eine größere Schonung des gesunden Gewebes erreicht. Diese Ausführungsform soll als "Kreuzfeuerbeschallung" bezeichnet werden.

Eine kontinuierliche Änderung der Beschallungsrichtung während der Applikation, unter Beibehaltung des Zielvolumens ("Pendelbeschallung") stellt eine weitere vorteilhafte Ausführungsform der Erfindung dar.

Zur Beschallung wird ein Beschallungsplan erstellt. Aus der Kenntis der Druckverteilung beim Einzelschuss $i:= (1,........,N)$, läßt sich eine kummulierte Druckverteilung bei N-Schüssen berechnen. Diese kummulierte Druckverteilung, im folgenden "Dosis" genannt, kann in erster Näherung wie folgt beschrieben werden:

$$\text{Dosis} = \sum_{i=1}^{N} p_i (X,Y,Z) \quad \text{wobei}$$

N die Anzahl der Schüsse und $p_i (X,Y,Z,)$ den Druck am Ort $(X,Y,Z)$ beim i-ten-Schuss beschreibt.

–4–

Für die Berechnung der räumlichen Druckverteilung soll eine Beschallungsplanung erstellt werden.

Die Erfindung wird nachfolgend anhand von Figuren näher erläutert. Es zeigen:

Fig.1 schematische Darstellung einer pathologischen Neubildung mit umgebendem gesunden Gewebe.

Fig.2 bis 4 die fortschreitende Veränderung der Endothelwand der Kapillaren bei Stoßwellenbehandlung und

Fig.5 eine Einrichtung zur Stoßwellenthromboisierung und deren Anwendung.

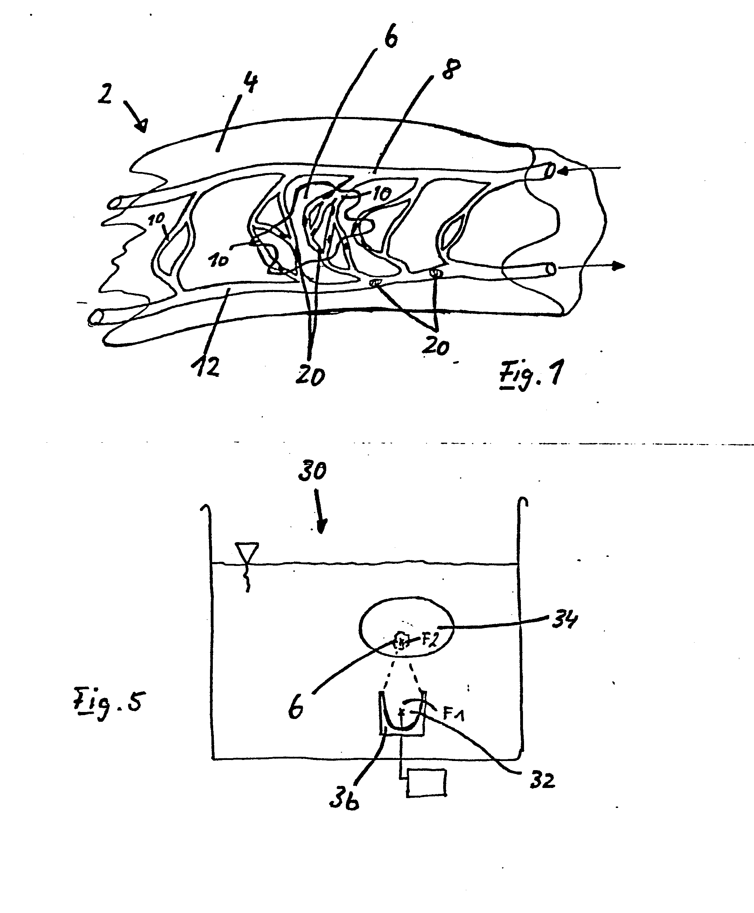

Fig.1 zeigt schematisch einen Gewebsausschnitt 2, der aus gesundem Gewebe 4 und einer pathologischen Neubildung (Tumor) 6 besteht. Der Tumor wird von der Arteriolen 8 versorgt, die sich im Bereich des Tumors in Kapillaren 10 verästeln. Der Blutrückfluß erfolgt über Venolen 12.

Die Blutgefäße 10, die den Tumor 4 durchziehen, haben gegenüber gesunden Kapillaren dünnere Wände und bestehen nur aus einer Endothelschicht. Der Durchmesser solcher Kapillaren beträgt ungefähr 8 μm, wie in Fig.2 gezeigt ist. Die aus platten, faßdaubenartig aneinandergereihten Endothelzellen 14 mit Zellkern 16 bestehende Kapillare wird einer fokussierten Stoßwelle ausgesetzt. Dabei schwellen die Endothelzellen zunächst an (Fig. 3) und die Querschnittsfläche 18 für das Blut wird verkleinert. Bei serieller Beschallung mit höherer Energie kommt es schließlich zu einer irreversiblen Veränderung der die Kapillarenwand bildenden Endothelschicht; dies ist in Fig 4 gezeigt. Die Kapillare kann ihrer Funktion nicht mehr nachkommen, Erythrozythen treten ins Gewebe aus und die Versorgung des Tumors 6 mit Blut wird unterbunden bzw. gehemmt. Der Druck im Fokus hat bei Versuchen 1000 bar bis 2000 bar betragen.

In Fig.1 sind dunkle Punkte 20 gezeigt, die das Auftreten von Thromboisierungen bei Stoßwellenbehandlung verdeutlichen sollen. An diesen Punkten ist die Versorgung des Tumors mit Blut blockiert bzw. teilblockiert, sodaß er nicht mehr ausreichend versorgt wird und Wachstumshemmung auftritt.

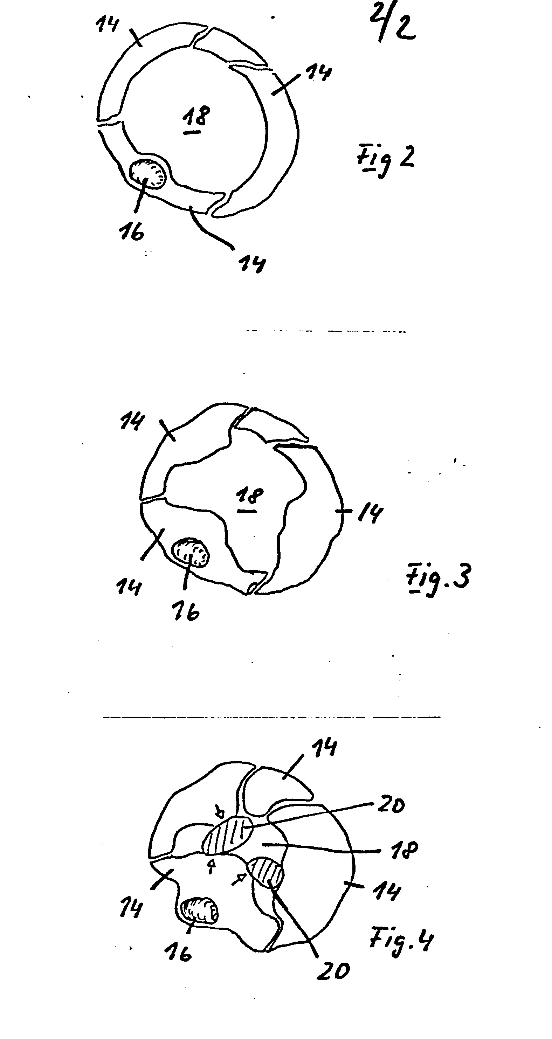

Fig.5 zeigt eine Einrichtung zur Durchführung des Verfahrens in prinzipieller Darstellung. Innerhalb einer wassergefüllten Wanne 30 befinden sich eine Stoßwellenquelle 32 und ein Patient 34. Die Stoßwellenquelle befindet sich im Ausführungsbeispiel im Brennpunkt F1 eines Rotationsellipsoids 36. Von der Stoßwellenquelle ausgehende Stoßwellen werden im Fokus F2 konzentriert. Dort befindet sich das zu behandelnde Gewebe.

Zur Erzeugung von Stoßwellen können Funkenentladung, Laser, elektromechanische Quellen oder piezoelektrische Erzeuger verwendet werden. Zur Fokussierung können an Stelle eines Ellipsoiden auch Kugelkalotten oder Linsenfokussierung dienen.

Zur Pendelbeschallung kann sich das Ellipsoid 3b mit Stosswellenquelle 32 pendelnd um F2 bewegen oder der Körper des Patienten 34 kann bei stillstehendem Ellipsoid Pendelbewegungen um F2 ausführen.

13.10.86
PaL/mr

DORNIER MEDIZINTECHNIK GMBH
Postfach 1128
8034 Germering 1

0226740

Reg. M 105 EU

PATENTANSPRÜCHE

1. Verfahren zur spezifischen Hemmung des Wachstums von pathologischen Neubildungen im lebenden Organismus, dadurch gekennzeichnet, daß fokussierte Stoßwellen, deren Energie unterhalb des Zerstörungseffektes der Zellen liegt, auf die die pathologische Neubildung versorgenden Blutgefäße einwirken und diese thromboisieren.

2. Verfahren nach Anspruch 1, dadurch gekennzeichnet, daß die Beaufschlagung der die pathologische Neubildung versorgenden Blutgefäße aus verschiedenen Richtungen und somit als "Kreuzfeuerbeschallung" erfolgt.

3. Verfahren nach Anspruch 1 bis 3, dadurch gekennzeichnet, daß die Beaufschlagung unter kontinuierlicher Änderung der Beschallungsrichtung unter Beibehaltung des Zielvolumens und somit als "Pendelbeschallung" erfolgt.

4. Vorrichtung zur Durchführung des Verfahrens nach Ansprüchen 1 - 3, dadurch gekennzeichnet, dass sich innerhalb einer wassergefüllten Wanne eine rotationselliptische Fokussierungskammer mit einer Stosswellenquelle in ihrem Brennpunkt F 1 und sich das zu behandelnde Gewebe im Körper eines Patienten im Brennpunkt F 2 befindet.

13.10.86
PaL/mr

2

4

6

8

10

10

10

12

20

20

20

_Fig.1_

30

34

F2

6

F1

_Fig.5_

36

32

Fig 2

Fig.3

Fig.4

# EUROPÄISCHER TEILRECHERCHENBERICHT,

der nach Regel 45 des Europäischen Patentübereinkommens für das weitere Verfahren als
europäischer Recherchenbericht gilt

**0226740**
Nummer der Anmeldung

EP 86 11 4175

Europäisches
Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 090 138 (DORNIER GmbH)<br>* Figur 1; Seite 3, Zeilen 10-18 *<br>-------------- | 4 | A 61 B 17/22 |

**RECHERCHIERTE SACHGEBIETE (Int. Cl.4)**

A 61 B

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: **4**

Unvollständig recherchierte Patentansprüche:

Nicht recherchierte Patentansprüche: **1-3**

Grund für die Beschränkung der Recherche:

Verfahren zur chirurgischen oder
therapeutischen Behandlung des
menschlichen oder tierischen Körpers
(Siehe Art. 52(4) des Europäischen
Patentübereinkommens).

| Recherchenort | Abschlußdatum der Recherche | Prüfer |
|---|---|---|
| Den Haag | 20-03-1987 | NEILL |